Europäisches Patentamt

European Patent Office

Office européen des brevets

(19)

(11) Veröffentlichungsnummer : **0 124 184**
**B1**

(12) ## EUROPÄISCHE PATENTSCHRIFT

(45) Veröffentlichungstag der Patentschrift :
15.04.87

(21) Anmeldenummer : 84200606.6

(22) Anmeldetag : 02.05.84

(51) Int. Cl.⁴ : **G 21 K 1/02,** A 61 B 6/06,
G 03 B 42/02

(54) Vorrichtung zur Beseitigung von Streustrahlung.

(30) Priorität : 03.05.83 DE 3316003

(43) Veröffentlichungstag der Anmeldung :
07.11.84 Patentblatt 84/45

(45) Bekanntmachung des Hinweises auf die Patenterteilung : 15.04.87 Patentblatt 87/16

(84) Benannte Vertragsstaaten :
BE DE FR GB NL

(56) Entgegenhaltungen :
DE-A- 2 733 613
DE-B- 1 049 226
FR-A- 1 104 530
FR-A- 1 147 221
US-A- 2 846 588

(73) Patentinhaber : Philips Patentverwaltung GmbH
Billstrasse 80
D-2000 Hamburg 28 (DE)
DE
N.V. Philips' Gloeilampenfabrieken
Groenewoudseweg 1
NL-5621 BA Eindhoven (NL)
BE FR GB NL

(72) Erfinder : Bauer, Manfred
Heinrichstrasse 8a
D-2077 Brunsbek 2 (DE)

(74) Vertreter : Hartmann, Heinrich, Dipl.-Ing. et al
Philips Patentverwaltung GmbH Billstrasse 80 Postfach 10 51 49
D-2000 Hamburg 28 (DE)

Jouve, 18, rue St-Denis, 75001 Paris, France

## Beschreibung

Die Erfindung betrifft eine Vorrichtung für ein Röntgengerät zur Beseitigung von Streustrahlung mittels eines Streustrahlenrasters, das mit einer Rolle gekoppelt ist, die durch eine Kraft gegen einen Antriebskörper gedrückt wird, dessen Oberfläche so geformt ist, daß bei einer Drehung des Antriebskörpers der Berührungspunkt mit der Rolle in Richtung senkrecht zu deren Drehachse verschoben wird. Eine solche Vorrichtung ist aus der DE-OS 27 33 613 bekannt. Bei der bekannten Vorrichtung wird durch Drehung des Antriebskörpers das Streustrahlenraster senkrecht zur Richtung seiner Lamellen verschoben, damit diese während einer Röntgenaufnahme nicht mit abgebildet werden.

Die Verschiebung muß am Anfang einer Aufnahme — deren Dauer bei Verwendung eines Belichtungsautomaten nicht im voraus abgeschätzt werden kann — relativ schnell erfolgen, damit auch bei kurzen Aufnahmen solche Abbildungen vermieden werden. Da der maximale Verschiebungsweg — der sogenannte Hub — bei derartigen Vorrichtungen in der Regel aus konstruktiven Gründen begrenzt ist, wird bei vielen bekannten Vorrichtungen zur Beseitigung der Streustrahlung das Streustrahlenraster hin und herbewegt. Dabei besteht aber die Gefahr, daß die Rasterlamellen in den Umkehrpunkten, in denen die Bewegungsrichtung umgekehrt wird, abgebildet werden, weil das Streustrahlenraster in den Umkehrpunkten still steht und das mehrmals während einer Aufnahme.

Bei der aus der DE-OS 27 33 613 bekannten Vorrichtung wird diese Gefahr dadurch beseitigt, daß die Rolle exzentrisch am Streustrahlenraster bzw. einem damit verbundenen Teil gelagert ist, so daß sich die Lage der Umkehrpunkte ändert. Diese Vorrichtung erfordert allerdings einen relativ schnellen Antrieb, wodurch die Umkehrpunkte während einer Aufnahme mehrmals erreicht werden. Dadurch kann das Röntgengerät in Schwingungen geraten, die sich nachteilig auf die Schärfe der Röntgenaufnahme auswirken können.

Es ist Aufgabe der vorliegenden Erfindung, eine Vorrichtung der eingangs genannten Art so auszugestalten, daß die Dauer des Stillstandes in einem Umkehrpunkt so weit herabgesetzt wird, daß die Gefahr einer wahrnehmbaren Abbildung der Lamellen weitgehend beseitigt ist. Diese Aufgabe wird dadurch gelöst, daß die Rolle um ihr Zentrum drehbar mit einem Hebel verbunden ist, der um eine zur Drehachse der Rolle parallele Achse schwenkbar mit den Streustrahlenraster oder einem damit verbundenen Teil verbunden ist, daß der Abstand zwischen der Schwenkachse und der Drehachse gleich dem Radius der Rolle ist oder nur geringfügig größer als dieser, und daß die Oberfläche des Antriebskörpers so geformt ist, daß sich ihre Steigung in dem Umkehrpunkt, in dem die Rolle am weitesten entgegen der Kraftrichtung verschoben ist, unstetig ändert.

Die Erfindung basiert auf folgenden Überlegungen :

Die mit der Rolle zusammenwirkende Oberfläche des Antriebskörpers ist so geformt, daß sich in dem definierten Umkehrpunkt die Steigung unstetig ändert, d. h. die Oberfläche weist in diesem Umkehrpunkt eine Spitze auf. Wenn das Streustrahlenraster der Kontur dieses Antriebskörpers direkt folgen könnte, würde sich der gewünschte Verlauf mit kurzer Stillstandszeit im Umkehrpunkt ergeben. Jedoch beschreibt das Zentrum der auf der Oberfläche des Antriebskörpers ablaufenden Rolle aufgrund des endlichen Rollendurchmessers eine abgerundete Bahn ; wenn das Streustrahlenraster dieser abgerundeten Bahn folgen würde, ergäbe sich in den Umkehrpunkten eine relativ lange Stillstandszeit.

Dies wird bei der Erfindung jedoch dadurch vermieden, daß das Streustrahlenraster nicht direkt mit der Drehachse der Rolle verbunden ist, sondern mit der Schwenkachse des Hebels, an dem die Rolle gelagert ist. Da auf das Streustrahlenraster eine z. B. durch eine Feder erzeugte Kraft einwirkt, durch die die mit dem Streustrahlenraster über den Hebel gekoppelte Rolle gegen den Antriebskörper gedrückt wird, stellt sich die Verbindungslinie zwischen der Drehachse der Rolle und der Schwenkachse des Hebels stets senkrecht zur Oberfläche des Antriebskörpers ein. Wenn sich nun die Neigung der Oberfläche im Umkehrpunkt unstetig ändert, ändert sich beim Überfahren des Umkehrpunktes durch die Rolle abrupt die Winkelstellung der Verbindungsgeraden zwischen der Schwenkachse und der Drehachse der Rolle und damit auch die Lage der Schwenkachse, die die jeweilige Position des Streustrahlenrasters festlegt. Dadurch kann die Schwenkachse — und damit das Streustrahlenraster — unmittelbar der Kontur des Antriebskörpers folgen, obwohl die Drehachse der Rolle aufgrund des endlichen Rollendurchmessers dies nicht vermag.

Die Erfindung wird nachstehend anhand der Zeichnungen näher erläutert. Es zeigen

Figur 1 eine perspektivische Ansicht des Streustrahlenrasterantriebes,

Figur 2 eine Abwicklung der Oberfläche des Antriebskörpers,

Figur 3 eine Einzelheit der Antriebseinrichtung in perspektivischer Darstellung,

Figur 4 eine seitliche Darstellung und

Figur 5 erläutert das Verhalten der Rolle im Bereich des Umkehrpunktes.

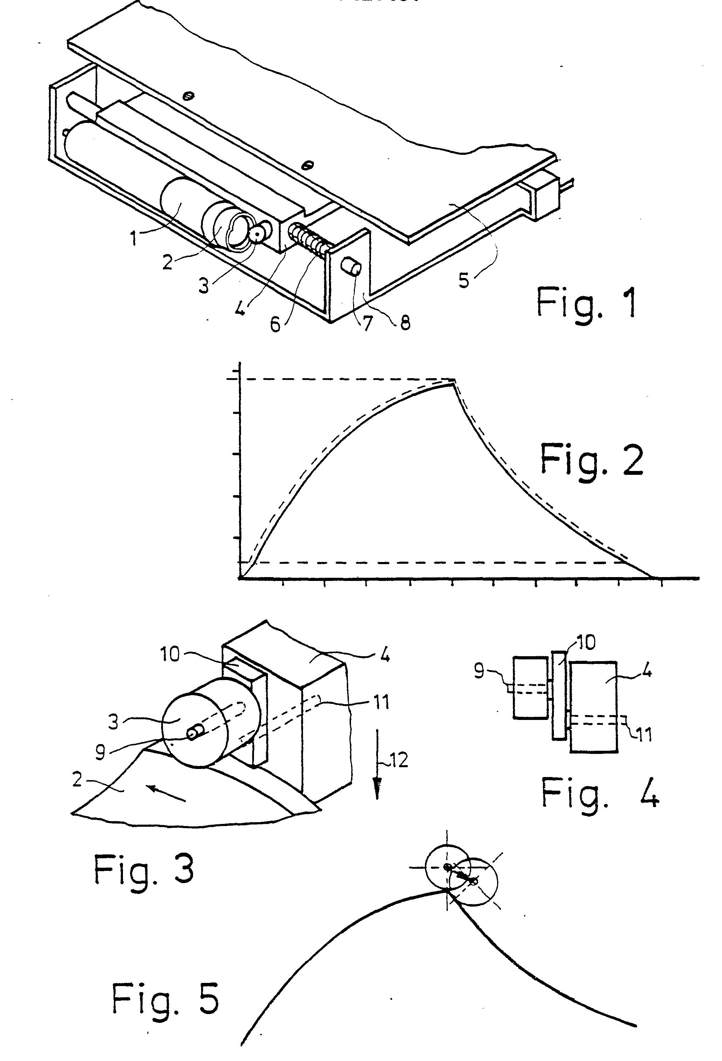

In Fig. 1 ist mit 1 ein Getriebemotor bezeichnet, der einen Antriebskörper mit ringförmigem Querschnitt in eine Drehbewegung mit konstanter Winkelgeschwindigkeit versetzt. Mit der Stirnfläche des Antriebskörpers 2 steht eine Rolle 3 in Berührung, deren Drehachse senkrecht zur Drehachse des Antriebskörpers 2 verläuft und die mit einem Teil 4 gekoppelt ist. das fest mit dem Streustrahlenraster 5 verbunden ist, dessen nicht näher dargestellte Lamellen senkrecht zur Drehachse des Antriebskörpers 2 verlaufen. Der Teil 4, mit dem die Rolle 3 gekoppelt ist, ist auf einer Führungsstange 7 geführt, die parallel zur Drehachse des Antriebskörpers 2 verläuft und auf der eine Feder 6 angeordnet ist, die auf das Teil 4 drückt, was zur Folge hat, daß die mit diesem Teil gekoppelte Rolle 3 gegen die Stirnfläche des Antriebskörpers 2 gedrückt wird. Die Bauteile sind an einem Träger 8 einander funktionsgerecht zugeordnet und befestigt. Die in Fig. 1 dargestellte Einheit eignet sich insbesondere für Mammographieaufnahmen.

Fig. 2 zeigt eine Abwicklung der Stirnfläche des Antriebskörpers bzw. die Position eines Punktes auf der Stirnfläche des Antriebskörpers (gemessen in Richtung der Drehachse dieses Körpers) als Funktion des Drehwinkels. Der Beginn der Drehbewegung erfolgt immer in derjenigen Position des Antriebskörpers, in der das Teil 4 durch die Feder 6 am weitesten in Richtung auf den Motor 1 gedrückt ist, d. h. sie beginnt am unteren Umkehrpunkt. Wie aus Fig. 2 ersichtlich, steigt die Kurve zunächst relativ steil, um dann immer flacher zu werden. Der steile Anstieg der Kurve zu Beginn soll sicherstellen, daß bei kurzzeitigen Aufnahmen, bei denen nur ein Teil der Kurve durchlaufen wird, das Streustrahlenraster so schnell bewegt wird, daß seine Lamellen auf der Röntgenaufnahme nicht abgebildet werden. Die dann immer flacher werdende Kurve erreicht schließlich ein Maximum in dem oberen Umkehrpunkt, in dem die Rolle am weitesten entgegen der Kraft verschoben ist. Von da an fällt die Kurve abrupt mit zunächst größer und allmählich kleiner werdender Steigung ab, bis wieder die Ausgangslage erreicht ist. Durch diese Gestaltung der Stirnfläche des Antriebskörpers 2 ergibt sich im Bereich des Maximums eine unstetige Änderung der Steigung.

Wie aus Fig. 3 hervorgeht, ist die Rolle 3 nicht direkt mit dem Teil 4 verbunden ; vielmehr wird ihre Drehachse 9 von einem Hebel 10 getragen, der um die zur Drehachse 9 parallele Achse 11 schwenkbar an dem Teil 4 gelagert ist. Wie insbesondere aus Fig. 4 hervorgeht, entspricht der Abstand zwischen der Drehachse 9 der Rolle 3 und der Schwenkachse 11 des Hebels gerade dem Radius der Rolle. Da außerdem die Federkraft auf das Teil 4 einwirkt, die durch den Vektor 12 symbolisiert ist, nimmt der Hebel immer eine solche Stellung ein, daß die Verbindungsgerade zwischen den beiden Achsen senkrecht zur Oberfläche des Antriebskörpers 2 verläuft. Infolgedessen folgt die Schwenkachse 11 — und damit auch der Teil 4 bzw. das Streustrahlenraster 5 — der Kontur der Stirnfläche des Antriebskörpers. Daran ändert sich auch nichts, wenn die Rolle die Spitze (und das Streustrahlenraster den Umkehrpunkt) erreicht und übersc hreitet. Da sich dann jedoch die Steigung der die Rolle berührenden Fläche des Antriebskörpers sprungartig ändert, ändert sich auch, wie insbesondere aus Fig. 5 hervorgeht, sprungartig die Lage der Drehachse der Rolle, und diese bewegt sich anschließend ebenso wie die Hebelachse 11 entsprechend dem Kurvenverlauf abwärts und endet im unteren Umkehrpunkt. Da der obere Umkehrpunkt dabei von der Rolle nahezu sprungartig passiert wird, ist eine Abbildung in diesem Punkt weitgehend ausgeschlossen.

Mit der erfindungsgemäßen Vorrichtung ist es also möglich, eine einmal hin- und hergehende Bewegung zu erzeugen, die bei einem Hub von knapp 10 mm (Abstand zwischen dem oberen und dem unteren Umkehrpunkt) und einer Dauer der Umdrehung des Antriebskörpers von 5 sec genügend schnell ist, um eine ausreichende Verwischung zu erzeugen.

In Fig. 1 ist als Antriebskörper ein Hohlzylinderkörper dargestellt. Jedoch ist es auch grundsätzlich möglich, stattdessen eine Scheibe zu verwenden, die um eine zur Rollenachse parallele Achse gedreht wird und deren am weitesten von ihrer Drehachse entfernter Bereich, der den oberen Umkehrpunkt des Rasters bestimmt, eine unstetige Änderung seines Tangentenwinkels aufweist. Diese Bauform ist im allgemeinen jedoch etwas weniger kompakt als die in Fig. 1 dargestellte, weil der Motor dann um 90° versetzt montiert werden muß.

**Patentansprüche**

1. Vorrichtung für ein Röntgengerät zur Beseitigung von Streustrahlung mittels eines Streustrahlenrasters, das mit einer Rolle gekoppelt ist, die durch eine Kraft gegen einen Antriebskörper gedrückt wird, dessen Oberfläche so geformt ist, daß bei einer Drehung des Antriebskörpers der Berührungspunkt mit der Rolle in Richtung senkrecht zu deren Drehachse verschoben wird, dadurch gekennzeichnet, daß die Rolle (3) um ihr Zentrum drehbar mit einem Hebel verbunden ist, der um eine zur Drehachse (9) der Rolle parallele Achse (11) schwenkbar mit dem Streustrahlenraster (5) oder einem damit verbundenen Teil (4) verbunden ist, daß der Abstand zwischen der Schwenkachse und der Drehachse gleich dem Radius der Rolle ist oder nur geringfügig größer als dieser, und daß die Oberfläche des Antriebskörpers (2) so geformt ist, daß sich ihre Steigung in dem Umkehrpunkt, in dem die Rolle (3) am weitesten entgegen der Kraftrichtung verschoben ist, unstetig ändert.

2. Vorrichtung nach Anspruch 1, dadurch gekennzeichnet, daß der Antriebskörper ein Hohlzylinder (2) ist, dessen der Rolle (3) zugewandte Stirnfläche ihre Neigung in dem Umkehrpunkt unstetig ändert.

**Claims**

1. A device for an X-ray apparatus for eliminating secondary radiation by means of a scatter grid which is coupled to a roller which is pressed by a force against a drive member whose surface is shaped so that during a rotation of the drive member, the point of contact with the roller is shifted in the direction perpendicular to its rotary shaft, characterized in that the roller (3) is connected to a lever so as to be rotatable about its centre, said lever being connected to the scatter grid (5), or to a part (4) which is connected thereto, so as to be pivotable about a shaft (11) which is parallel to the rotary shaft (9) of the roller, the distance between the pivot shaft and the rotary shaft being equal to or only slightly larger than the radius of the roller, the surface of the drive member (2) being shaped so that in the reversal point in which the roller (3) has been displaced furthest against the direction of the force, its rise changes non-uniformly.

2. A device as claimed in Claim 1, characterized in that the drive member is formed by a hollow cylinder (2) whose end face which faces the roller (3) changes its inclination non-uniformly at the reversal point.

**Revendications**

1. Dispositif pour un appareil à rayons X destiné à éliminer le rayonnement dispersé au moyen d'une grille antidiffusante qui est couplée à un galet qui est pressé par une force contre un organe d'entraînement dont la surface est conformée d'une manière telle que, lors d'une rotation de l'organe d'entraînement, le point de contact avec le galet soit déplacé dans une direction perpendiculaire à son axe de rotation, caractérisé en ce que le galet (3) est relié à rotation autour de son centre à un levier qui est relié de manière à pouvoir pivoter autour d'un axe parallèle à l'axe de rotation du galet à la grille antidiffusante ou à une pièce reliée à cette grille, que la distance entre l'axe de pivotement et l'axe de rotation est égale au rayon du galet ou n'est que légèrement supérieure à celle-ci et que la surface de l'organe d'entraînement (2) est façonnée de manière que sa pente varie de manière discontinue au point de rebroussement, au niveau duquel le galet (3) est déplacé au maximum à l'encontre de la direction de la force.

2. Dispositif suivant la revendication 1, caractérisé en ce que l'organe d'entraînement est un cylindre creux (2) dont l'inclinaison de la face d'about tournée vers le galet (3) varie de manière discontinue au niveau du point de rebroussement.

Fig. 1

Fig. 2

Fig. 3

Fig. 4

Fig. 5